# EUROPEAN PATENT SPECIFICATION

(11) **EP 3 856 752 B1**
(45) Date of publication and mention of the grant of the patent: **29.04.2026**
(21) Application number: 19773682.0
(22) Date of filing: 11.09.2019
(51) Int. Cl.: C07F 17/00, C07C 49/623, C07C 49/633

(54) **SYNTHESIS OF SUBSTITUTED CYCLOPENTADIENE COMPOUNDS AND METALLOCENES**
SYNTHESE VON SUBSTITUIERTEN CYCLOPENTADIENVERBINDUNGEN UND METALLOCENEN
SYNTHÈSE DE COMPOSÉS DE CYCLOPENTADIÈNE SUBSTITUÉS ET DE MÉTALLOCÈNES

(30) Priority: 28.09.2018 US 201862737974 P
(43) Date of publication of application: 04.08.2021
(73) Proprietor: Dow Global Technologies LLC, Midland, MI 48674 (US)
(72) Inventor: PADILLA-ACEVEDO, Angela I., Lake Jackson, Texas 77566 (US)
(74) Representative: Boult Wade Tennant LLP
(86) International application number: PCT/US2019/050546
(87) International publication number: WO 2020/068420

(56) References cited:
- EP-A1- 0 780 395
- EP-A2- 0 693 506
- WO-A1-2019/067271
- WO-A1-2019/067273
- BRAUDE, E. A. ET AL: "Polycyclic systems. V. New route to azulenes", JOURNAL OF THE CHEMICAL SOCIETY, 1 January 1953 (1953-01-01), pages 2208 - 2216, XP002795463, ISSN: 0368-1769, DOI: 10.1039/JR9530002208
- CONIA J M ET AL: "Sur la préparation de cyclopenténones par action de l'acide polyphosphorique sur les esters d'acides alpha-éthyléniques", BULLETIN DE LA SOCIETE CHIMIQUE DE FRANCE, SOCIETE FRANCAISE DE CHIMIE. PARIS, FRANCE, 1 January 1970 (1970-01-01), pages 2981 - 2991, XP002430352, ISSN: 0037-8968
- YANG, QING ET AL: "Novel and efficient syntheses of bis(.eta.5-tetrahydroindenyl) dichlorides of titanium, zirconium and hafnium", SYNLETT, vol. 6, 1 June 1996 (1996-06-01), pages 563 - 564, XP002795464, ISSN: 0936-5214, DOI: 10.1055/S-1996-5495 10.1055/S-1996-5495

## Description

### FIELD

Synthesizing substituted cyclopentadiene compounds, and substituted metallocenes therefrom.

### INTRODUCTION

Metallocene compounds comprise a transition metal atom that is bonded to two unsubstituted cyclopentadienyl ligands (Cp, formally an anion of formula [C₅H₅]⁻). Substituted metallocene compounds comprise a transition metal atom that is bonded to one Cp ligand and one substituted cyclopentadienyl ligand (substituted-Cp), which is isolobal to Cp, or a transition metal atom that is bonded to two independently selected substituted-Cp, which are isolobal to Cp. The transition metal is an element of any one of Groups 3 to 12 useful for catalyzing polymerizations of olefins. Examples of the transition metal are Group 4 metals such as titanium, zirconium, and hafnium. Examples of the substituted cyclopentadienyl ligands are methylcyclopentadienyl and 4,5,6,7-tetrahydroindenyl. A typical substituted metallocene compound is 4,5,6,7-tetrahydroindenylcyclopentadienyl zirconium dimethyl complex ((4,5,6,7-tetrahydroindenyl)(cyclopentadienyl)Zr(CH₃)₂). Typically, the synthesis of the substituted metallocene compound involves numerous synthetic steps, uses expensive reagents, and/or employs a hydrogenation catalyst and step (e.g., platinum-catalyzed hydrogenation step) to convert an indenyl-cyclopentadienyl zirconium dichloride compound to a 4,5,6,7-tetrahydroindenyl-cyclopentadienyl zirconium dichloride compound. See, e.g., US 2004/0249096 A1 and US 5,721,185.

Uemichi, Yoshio; Kanoh, Hisao. Kenkyu Hokoku-Asahi Garasu Kogyo Gijutsu Shoreikai, Volume 49, Pages 225-30, 1986. CODEN:AGKGAA. ISSN:0365-2599 report that platinum is especially potent source of polyethylene degradation. Uemichi, Yoshio; Makino, Yutaka; Kanazuka, Takaji, Degradation of polyethylene to aromatic hydrocarbons over metal-supported activated carbon catalysts, Journal of Analytical and Applied Pyrolysis (1989), 14(4), 331-44.
An article taken from Journal of the Chemical Society, 1 January 1953, pages 2208-2216 relates to new routes to azulenes.

See also the following. Tabatabaenian, K.; Mamaghani, M.; Neshat, A.; Masjedi, M. Synthesis and Spectroscopic Studies of New Substituted Dinuclear η5-4,5,6,7-Tetrahydroindenyl Ruthenium Complexes. Russian Journal of Coordination Chemistry. 2003, 29, 7, 501. Austin, R. N.; Clark, T. J.; Dickson, T. E.; Killian, C. M.; Nile, T. A.; Shabacker, D. J.; McPhail, T. A. Synthesis and Properties of Novel Substituted 4,5,6,7-tetrahydroindenes and Selected Metal Complexes. Journal of Organometallic Chemistry. 1995, 491, 11. Conia, J. M.; Leriverend, M. L. Tetrahedron Letters. 1968, 17. 2101 (Conia et al.). L. Rand and R. J. Dolinski, J. Org. Chem., 1966, 31, 3063 and L. Rand and R. J. Dolinski, J. Org. Chem., 1966, 31, 4061 (collectively "Rand and Dolinski"). Yokota, K.; Kohsaka, T.; Ito, K.; Ishihara, N. Consideration of Mechanism of Styrene/Ethylene Copolymerization with Half-Titanocene Catalysts. Journal of Polymer Science. 2005, 43, 5041. JP10316694A to Tetsuya, I., et. al. Brancaccio G.; Lettieri, G.; Monforte, P.; Larizza, A. Farmaco, Edizione Scientifica. 1983, 9, 702-8. Eaton, P. E.; Carlson, G. R.; Lee, J. T. Phosphorus Pentoxide-Methanesulfonic Acid. A Convenient Alternative to Polyphosphoric Acid. J.Org. Chem. 1978, 38, 4071. Paquette, L. A.; Stevens, K. E., Can. J. Chem. 1984, 62, 2415. Paquette, L. A.; Cheney, D. L., J. Org. Chem. 1989, 54, 3334. J.Org. Chem. 1966, 3065.

Conia, et al. reported that reacting cyclohexene and crotonic acid in presence of polyphosphoric acid (PPA) exclusively gave as a sole product 2,3,4,5,6,7-hexahydro-3-methyl-1 H-inden-1-one (structure 1 in Conia et al.). Conia et al. reported reacting cyclopentyl crotonate or cyclohexyl crotonate in the presence of PPA gave 3-methyl-bicyclo[3.3.0]-2-octen-1-one (40% yield, Table 1 in Conia et al.) or 2,3,4,5,6,7-hexahydro-3-methyl-1 H-inden-1-one (60% yield, Table 2 in Conia et al.), respectively.

Rand and Dolinski report that using polyphosphoric acid (PPA) or a mixture of phosphorous pentoxide (P₂O₅ or P₄O₁₀) and PPA to catalyze the reaction of a cycloheptene, cyclohexene, or cyclopentene with an alpha,beta-unsaturated carboxylic acid such as acrylic acid or crotonic acid gives a reaction mixture that contains or is free of an ester by-product such as cycloheptyl crotonate, cyclohexyl crotonate, or cyclopentyl crotonate. Relatively how much of the ester by-product is made is said to depend on the amount of phosphorous pentoxide used in the mixture with PPA or the amount of the PPA or P₂O₅/PPA mixture relative to the amount of cycloalkene.

### SUMMARY

We discovered cyclopentenone compounds ("cyclopentenones") are useful intermediates for making substituted cyclopentadiene compounds, which are useful for making metallocene catalysts, which are useful for polymerizing olefin monomers such as ethylene, propylene, alpha-olefins, and butadiene to make polyolefin polymers, which have a wide variety of industrial uses.

The present invention includes a number of embodiments. A method of synthesizing a substituted cyclopentadiene compound. The synthesis comprises a step of cyclizing, in the presence of a phosphorous pentoxide/methanesulfonic acid reagent (e.g., Eaton's reagent), an alpha,beta-unsaturated carboxylic acid, cycloalkyl ester compound to make a substituted cyclopentenone compound, and converting the substituted cyclopentenone compound to the substituted cyclopentadiene compound. Also, a method of synthesizing a substituted metallocene compound comprising a metal-(substituted cyclopentadienyl ligand) complex, wherein the substituted cyclopentadienyl ligand is made from the substituted cyclopentadiene compound. A metal-(substituted cyclopentadienyl ligand) complex and substituted metallocene compound made by the method. A substituted metallocene catalyst made from the metal-(substituted cyclopentadienyl ligand) complex or substituted metallocene compound.

We discovered a shorter synthesis of substituted cyclopentadiene compounds. The synthesis uses a phosphorous pentoxide/methanesulfonic acid reagent, and may be run at a lower temperature and yet be higher yielding than a prior PPA-based synthesis of Conia et al. Also, the synthesis avoids using a hydrogenation catalyst, a hydrogenation step, and a hydrogenation catalyst filtration step. Therefore, the inventive metal-(substituted cyclopentadienyl ligand) complex and substituted metallocene catalyst made therefrom, and polyolefins made therewith are beneficially free of (added) hydrogenation catalyst metals such as platinum, palladium, nickel, rhodium, and ruthenium. As discussed above, polyolefin degradation problems have been attributed to hydrogenation catalyst metals are reported in the literature, and thus the inventive complex and catalyst beneficially avoid any such problem(s).

### DETAILED DESCRIPTION

Certain inventive embodiments are described below as numbered aspects for easy cross-referencing. Additional embodiments are described elsewhere herein.

Aspect 1. A method of synthesizing a substituted cyclopentadiene compound, the method comprising (A) contacting a compound of formula (1) ("compound (1)"): 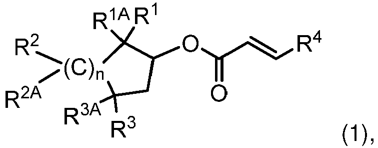 wherein subscript n is 1, 2, 3, or 4; and each of groups R1, R1A, R2, R2A, R3, R3A, and R4 is independently H or (C₁-C₄)alkyl, or any two adjacent R1 to R3 groups are bonded together to form a (C₁-C₄)alkylene and the remaining groups of R1 to R3A is H or (C₁-C₄)alkyl, with an effective amount of a phosphorous pentoxide/methanesulfonic acid reagent (P₂O₅/H₃CSO₃H reagent) and under reaction conditions sufficient to make a compound of formula (2) ("compound (2)"): 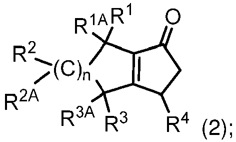 wherein subscript n and groups R1 to R4 are as defined above; and with the proviso that the contacting step (A) is free of added polyphosphoric acid (PPA); (B) contacting the compound (2) with a metal-R5 reducing agent that is either a hydride-functional reducing agent or a (C₁-C₄)alkyl lithium, under reaction conditions sufficient to make a compound of formula (3) ("compound (3)"): 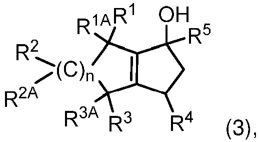 wherein subscript n and groups R1 to R4 are as defined above and R5 is H or (C₁-C₄)alkyl, respectively; and (C) contacting the compound (3) with dehydration reaction conditions to make a substituted cyclopentadiene compound of formula (4) ("compound (4)"): 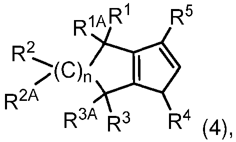 wherein subscript n and groups R1-R5 are as defined above. When the metal-R5 reducing agent is the hydride-functional reducing agent, R5 is H in compound (3). When the metal-R5 reducing agent is the (C₁-C₄)alkyl lithium, R5 is (C₁-C₄)alkyl in compound (3). The reaction conditions sufficient to make compound (2) include an anhydrous environment and a temperature from -80° to 30° C. Step (A) may be free of PPA.

Aspect 2. A method of synthesizing a substituted metallocene compound comprising a metal-(substituted cyclopentadienyl ligand) complex, the method comprising synthesizing the compound (4) according to steps (A) to (C) of aspect 1; (D) contacting the compound (4) with an alkyl lithium under reaction conditions sufficient to make a compound of formula (5) ("compound (5)"): 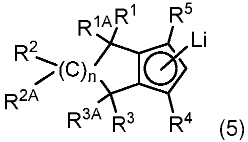 ; and (E) contacting the compound (5) with a compound of formula (6) ("compound (6)"): 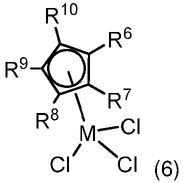 under reaction conditions sufficient to make a compound of formula (7) ("compound (7)"): 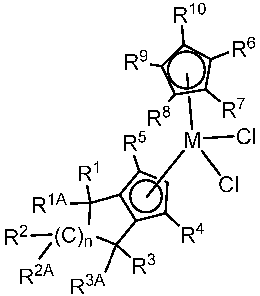 (7), wherein subscript n and groups R1 to R5 are as defined above; metal M is a metal of Group 4 of the Periodic Table of the Elements; and each of R6 to R8 is independently H or (C₁-C₄)alkyl and R9 and R10 is independently H or (C₁-C₄)alkyl or R9 and R10 are bonded together and are a (C₃-C₅)alkylene. In some embodiments M is Ti, Zr, or Hf; alternatively Zr or Hf; alternatively Ti or Zr; alternatively Ti or Hf; alternatively Ti; alternatively Zr; alternatively Hf.

Aspect 3. A method of synthesizing a zirconocene dimethyl complex, the method comprising synthesizing the compound (7) according to steps (A) to (E) of aspect 2, wherein M is Zr; and (F) contacting the compound (7) with an effective amount of methyl magnesium bromide under reaction conditions sufficient to make a compound of formula (8) ("compound (8)"): 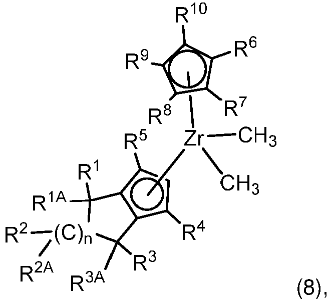 wherein subscript n and groups R1 to R10 are as defined above.

Aspect 4. The method of any one of aspects 1 to 3, wherein the ratio of P₂O₅ to H₃CSO₃H used to make the P₂O₅/H₃CSO₃H reagent is from 0.05/1 to 1/1 (weight/weight).

Aspect 5. The method of any one of aspects 1 to 4, wherein the ratio of P₂O₅ to H₃CSO₃H used to make the P₂O₅/H₃CSO₃H reagent is 0.1/1 (weight/weight). Known as Eaton's reagent.

Aspect 6. The method of any one of aspects 1 to 5, wherein the compound (4) is selected from the group consisting of any one of compounds (4a) to (4e): compound (4a) is compound (4) wherein subscript n is 2, R1 to R3A is H and each of R4 and R5 is methyl; compound (4b) is compound (4) wherein subscript n is 1 and R1-R4 are H and R5 is methyl; compound (4c) is compound (4) wherein subscript n is 1 and R1-R3A are H and each of R4 and R5 is methyl; compound (4d) is compound (4) wherein subscript n is 3 and R1-R3A are H and each of R4 and R5 is methyl; and compound (4e) is compound (4) wherein subscript n is 4 and R1, R1A, two R2, and each R2A are H and two R2 and each of R3, R4 and R5 is methyl. The method of any one of aspects 1 to 5, wherein any one of compounds (4a) to 4(e) is used to make a corresponding embodiment of compound (5) (designated as any one of compounds (5a) to (5e), respectively). The method of any one of aspects 1 to 5, wherein any one of compounds (5a) to (5e) plus compound (6) is used to make a corresponding embodiment of compound (7) (designated as any one of compounds (7a) to (7e), respectively). The method of aspect 3, wherein any one of compounds (7a) to (7e) is used to make a corresponding embodiment of compound (8) (designated as any one of compounds (8a) to (8e), respectively). The method of aspect 2 or 3, wherein compound (7) is any one of compounds (7f) and (7g):

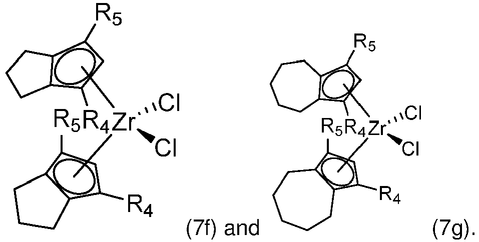

A method of making a dichloro compound of formula (9) can be provided: 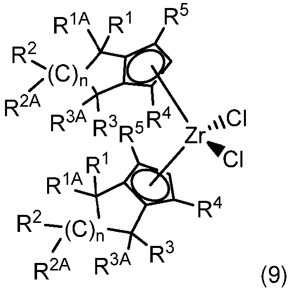 ("compound (9)"), wherein each subscript n independently is 1, 2, 3, or 4; and each of groups R1, R1A, R2, R2A, R3, R3A, R4, and R5 is independently H or (C₁-C₄)alkyl, or any two adjacent R1 to R3 groups are bonded together to form a (C₁-C₄)alkylene and the remaining group of R1 to R5 is H or (C₁-C₄)alkyl, the method comprising contacting two mole equivalents of the compound (5) with one mole equivalent of ZrCl₄, under reaction conditions sufficient to make the compound (9).

The method as defined above further comprising a step of contacting the compound (9) with an effective amount of methyl magnesium bromide under reaction conditions sufficient to make a dimethyl compound (10) ("compound (10)"), which has a structure identical to that of compound (9) except wherein each Cl atom of compound (9) is replaced by a methyl group (CH₃) in compound (10).

A compound of formula (9) made by the method as defined above or compound (10) made by the method as defined above can be provided. In some aspects compound (9) is any one of compounds (9a) to (9e) and compound (10) is any one of compounds (10a) to (10e), wherein in compound (9a) and (10a) each subscript n is 2, R1 to R3A is H and each of R4 and R5 is methyl; wherein in compound (9b) and (10b) each subscript n is 1 and R1-R4 are H and R5 is methyl; wherein in compound (9c) and (10c) each subscript n is 1 and R1-R3A are H and each of R4 and R5 is methyl; wherein in compound (9d) and (10d) each rein subscript n is 3 and R1-R3A are H and each of R4 and R5 is methyl; and wherein in compound (9e) and (10e) each subscript n is 4 and R1, R1A, two R2, and each R2A are H and two R2 and each of R3, R4 and R5 is methyl.

A method of polymerizing an olefin can be provided, the method comprising synthesizing the compound (7) according to the method of aspect 2 or synthesizing the compound (8) according to the method of aspect 3; contacting the compound (7) or (8) with an activator to make a catalyst; and contacting ethylene and/or an alpha-olefin with the catalyst under conditions sufficient to make a polyolefin polymer comprising a polyethylene homopolymer, an ethylene/alpha-olefin copolymer, or a poly(alpha-olefin) homopolymer; wherein the method is free of platinum, palladium, nickel, rhodium, and ruthenium.

The polyolefin polymer made by the method as defined above and being free of platinum, palladium, nickel, rhodium, and ruthenium.

The term "free of" means contains no detectable presence of.

As subscript n and groups R1 to R4 are defined for compound (1), so they may be defined for compounds (2) to (8). As group R5 is defined for the metal-R5 reducing agent, so it may be defined for compounds (3) to (8). As groups R6 to R10 are defined for compound (6), so they may be defined for compounds (7) and (8).

In some aspects, any one of, alternatively each of compounds (1) to (8) is characterized by any one of limitations (i) to (xxi): (i) wherein at least one of R1 to R3 is a (C₁-C₄)alkyl or R4 is H; (ii) wherein each of R1 to R4 is H; (iii) wherein each of R1 to R3 is H and R4 is methyl; (iv) wherein in compounds (1) to (8) each of R1 and R2 is H and each of R3 and R4 is methyl; (v) wherein R1 and/or R2 is methyl and R3 is H; (vi) wherein R1 is methyl, R2 is 1-methylethyl (i.e., isopropyl), and R3 is H; (vii) wherein R1 is 1-methylethyl (i.e., isopropyl), R2 is methyl, and R3 is H; (viii) wherein R1 and R2 independently are (C₁-C₄)alkyl, R3 is H, and the stereochemistry of the carbon atom bonded to R1 is (R) and the stereochemistry to the carbon atom bonded to R2 is (S); (ix) wherein R1 and R2 independently are (C₁-C₄)alkyl, R3 is H, and the stereochemistry of the carbon atom bonded to R1 is (S) and the stereochemistry to the carbon atom bonded to R2 is (R); (x) both (vi) and (viii); (xi) both (vi) and (ix); (xii) both (vii) and (viii); (xiii) both (vii) and (ix); (xiv) wherein R5 is H; (xv) wherein R5 is methyl; (xvi) both (i) and (xiv) or (xv); (xvii) both (ii) and (xiv) or (xv); (xviii) both (iii) and (xiv) or (xv); (xix) both (iv) and (xiv) or (xv); (xx) both (v) and (xiv) or (xv); and (xxi) any two adjacent R1 to R3 groups are bonded together to form a (C₁-C₄)alkylene and the remaining group of R1 to R3 is H or (C₁-C₄)alkyl.

Compound (1) may be obtained from a commercial supplier or synthesized from starting materials suitable for making alpha,beta-unsaturated carboxylic acid, cycloalkyl esters. Examples of commercially available compound (1) are (1a) (2E)-2-butenoic acid, cyclohexyl ester; (1b) (2E)-2-butenoic acid, cyclopentyl ester (CAS 1195328-04-1); (1c) (2E)-2-butenoic acid, cycloheptyl ester (CAS 10555-39-2); and (1d) propenoic acid, cyclopentyl ester (CAS 16868-13-6). (2E)-2-butenoic acid is also known as (E)-crotonic acid. Unless stated otherwise herein, "crotonic acid" means (2E)-2-butenoic acid. In some embodiments compound (1) is any one of compounds (1a) to (1d), alternatively compound (1) is selected from the group consisting of any three of compounds (1a) to (1d); alternatively compound (1) is compound (1a), alternatively compound (1b), alternatively compound (1c), alternatively compound (1d).

Compound (1) may be readily synthesized by reacting a corresponding cycloalkanol of formula (a): 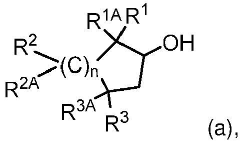 wherein subscript n and groups R1 to R3 are as defined for compound (1), with an alpha,beta-unsaturated carboxylic acid of formula (b): 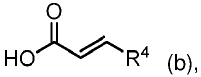 wherein R4 is as defined for compound (1), under dehydrating conditions. Suitable dehydrating conditions include refluxing toluene, a protic acid such as para-toluenesulfonic acid (pTsOH), and a Dean-Stark trap for removing, or a drying agent for sequestering, water that is generated. Examples of drying agent are 3 Ångström molecular sieves and anhydrous sodium sulfate. Methods and conditions for synthesizing carboxylic esters from the corresponding alcohol and carboxylic acid are well-known and useful. The compound (1) may also be synthesized by reacting the cycloalkanol of formula (a) with a corresponding alpha, beta-unsaturated carboxylic anhydride, which may be made by dehydrating two mole equivalents of compound (b).

Cycloalkanol compound (a) may be obtained from commercial suppliers or synthesized by well-known methods of making alcohols. Examples of commercially available compounds (a) wherein subscript n is 1 are (a1) cyclopentanol (CAS 96-41-3); (a2) 3-methyl-cyclopentanol (CAS 18729-48-1); (a3) 3,4-dimethyl-cyclopentanol (CAS 73316-51-5); and (a4) 3,3-dimethyl-cyclopentanol (CAS 60670-47-5). Examples of commercially available compounds (a) wherein subscript n is 2 are (a5) cyclohexanol (CAS 108-93-0); (a6) 2-methylcyclohexanol (mixture of stereoisomers or single enantiomers); (a7) 4-methylcyclohexanol (CAS 589-91-3); (a8) 2,5-dimethylcyclohexanol (CAS 3809-32-3); (a9) 5-methyl-2-(1-methylethyl)-cyclohexanol (e.g., as a mixture of stereoisomers or as any one enantiomer thereof such as (*1R,2S,5R*)-menthol). Examples of commercially available compounds (a) wherein subscript n is 3 are (a10) cycloheptanol (CAS 502-41-0); (a11) 4-methylcycloheptanol (CAS 90200-61-6); and (a12) 4,4-dimethylcycloheptanol (CAS 35099-84-4). Examples of commercially available compounds (a) wherein subscript n is 4 are (a13) cyclooctanol (CAS 696-71-9); and (a14) 3,5,7-trimethylcyclooctanol (CAS 1823711-29-0). In some embodiments compound (1) is made from, and the alcohol-derived portion containing R1-R3 corresponds to, any one of compounds (a1) to (a14), alternatively a compound selected from the group consisting of any thirteen of compounds (a1) to (a14), alternatively a compound (1) wherein subscript n is 1, 2, or 3; alternatively a compound (1) wherein subscript n is 1 or 2; alternatively a compound (1) wherein subscript n is 1; alternatively a compound (1) wherein subscript n is 2; alternatively a compound (1) wherein subscript n is 3 or 4; alternatively a compound (1) wherein subscript n is 3; alternatively a compound (1) wherein subscript n is 4.

Alpha,beta-unsaturated carboxylic acid compound (b) may be obtained from commercial suppliers or synthesized by well-known methods of making carboxylic acids. Examples of commercially available compounds (b) are (b1) acrylic acid (compound (b) wherein R4 is H); (b2) crotonic acid (compound (b) wherein R4 is methyl); (b3) 2-pentenoic acid (compound (b) wherein R4 is ethyl); and (b4) 2-hexenoic acid (compound (b) wherein R4 is propyl). In some embodiments compound (1) is made from, and the carboxylic acid-derived portion containing R4 corresponds to, any one of compounds (b1) to (b4); alternatively a compound selected from the group consisting of any three of compounds (b1) to (b4); alternatively compound (b1) or (b2); alternatively compound (b1); alternatively compound (b2); alternatively compound (b3) or (b4); alternatively compound (b3); alternatively compound (b4).

Activator (for activating compound (7) or (8) to form a catalyst). Also known as cocatalyst. Any metal containing compound, material or combination of compounds and/or substances, whether unsupported or supported on a support material, that can activate compound (8) to give a catalyst and an activator species. The activating may comprise, for example, abstracting at least one leaving group (e.g., at least one methyl) from the Zr of compound (8) to give the catalyst. The activator may be a Lewis acid, a non-coordinating ionic activator, or an ionizing activator, or a Lewis base, an alkylaluminum, or an alkylaluminoxane. The alkylaluminum may be a trialkylaluminum, alkylaluminum halide, or alkylaluminum alkoxide (diethylaluminum ethoxide). The trialkylaluminum may be trimethylaluminum, triethylaluminum ("TEAI"), tripropylaluminum, triisobutylaluminum, and the like. The alkylaluminum halide may be diethylaluminum chloride. The alkylaluminoxane may be a methyl aluminoxane (MAO), ethyl aluminoxane, or isobutylaluminoxane. The activator may be a MAO that is a modified methylaluminoxane (MMAO). The corresponding activator species may be a derivative of the Lewis acid, non-coordinating ionic activator, ionizing activator, Lewis base, alkylaluminum, or alkylaluminoxane, respectively. The activator species may have a different structure or composition than the activator from which it is derived and may be a by-product of the activation reaction. The metal of the activator typically is different than Group 4 metal such as zirconium. The molar ratio of metal content of the activator to zirconium content of compound (7) or (8) may be from 1000:1 to 0.5:1, alternatively 300:1 to 1:1, alternatively 150:1 to 1:1, alternatively 100.0:1 to 1:1.

Alkyl means an unsubstituted univalent saturated acyclic hydrocarbon that is straight chain (1 or more carbon atoms), branched chain (if 3 or more carbon atoms), or cyclic (if 3 or more carbon atoms). Each (C₁-C₄)alkyl is independently methyl, ethyl, propyl, 1-methylethyl, butyl, 1-methylpropyl, 2-methylpropyl, or 1,1-dimethylethyl. Alternatively each (C₁-C₄)alkyl is independently a (C₁-C₃)alkyl; alternatively a (C₂-C₄)alkyl; alternatively (C₁-C₂)alkyl; alternatively (C₂-C₃)alkyl; alternatively (C₃-C₄)alkyl; alternatively methyl or (C₃)alkyl. In some aspects each (C₁-C₄)alkyl is independently a (C₁-C₃)alkyl and each (C₁-C₃)alkyl is independently methyl, ethyl, propyl, or 1-methylethyl; alternatively methyl, propyl, or 1-methylethyl; alternatively methyl; alternatively ethyl; alternatively propyl; alternatively 1-methylethyl. Substituted alkyl is an alkyl as defined above except wherein one or more hydrogen atoms is formally replaced by a substituent such as unsubstituted alkyl, halogen, or alkylcarboxylic ester.

Alkyl lithium is a compound of formula alkyl-Li. Examples of alkyl lithium are methyl lithium, ethyl lithium, propyl lithium, n-butyl lithium, sec-butyl lithium, t-butyl lithium, and pentyl lithium. The (C₁-C₄)alkyl lithium is an alkyl lithium wherein the alkyl is methyl, ethyl, propyl, 1-methyl ethyl, butyl, 1-methylpropyl, 2-methylpropyl (sec-butyl), or 1,1-dimethylethyl (t-butyl).

Alkylene is unsubstituted divalent saturated acyclic hydrocarbon that is straight chain (1 or more carbon atoms), branched chain (if 3 or more carbon atoms), or cyclic (if 3 or more carbon atoms). Each (C₁-C₄)alkylene is independently methylene (CH₂), ethylene (CH₂CH₂), propylene (CH₂CH₂CH₂), 1-methylethylene (CH(CH₃)CH₂), butylene ((CH₂)₄), 1-methylpropylene (CH(CH₃)CH₂CH₂), 2-methylpropylene (CH₂CH(CH₃)CH₂), or 1,1-dimethylethylene (C(CH₃)₂CH₂. Substituted alkylene is an alkylene as defined above except wherein one or more hydrogen atoms is formally replaced by a substituent such as unsubstituted alkyl, halogen, or alkylcarboxylic ester.

Compound means a molecule or collection of molecules.

Dehydration reaction conditions include temperature and reagents effective for enhancing rate of loss of water from compound (3). Example of such reagents are 1 Molar (M) or higher hydrochloric acid (aqueous HCI) or anhydrous HCI in an organic solvent such as diethyl ether, ethanol, tetrahydrofuran or toluene. The hydrochloric acid may be from 1 M to 8 M, alternatively from 2 M to 6 M.

Effective amount is a quantity sufficient for enabling the making of a detectable amount of intended product (e.g., any one of compounds (2) to (8), as the case may be). An effective amount of the phosphoric and/or sulfonic acid reagent is a quantity thereof sufficient for enabling the making of a detectable amount of compound (2). Detectable amounts may be characterized by any suitable analytical method such as 1 H-nuclear magnetic resonance (1H-NMR), high performance liquid chromatography (HPLC, versus a known standard), gas chromatography (GC, versus a known standard), or mass spectrometry; typically 1H-NMR. The effective amount of the phosphorous pentoxide/methanesulfonic acid reagent used in step (A) may vary depending upon its composition, reaction conditions, and costs. A skilled person may determine an optimal effective amount thereof by starting with an initial reaction mixture of (1) and 95 wt% of the phosphorous pentoxide/methanesulfonic acid reagent, and thereafter systematically try reaction mixtures containing lower wt% of the phosphorous pentoxide/methanesulfonic acid reagent until an optimal result under the reaction conditions is found. The effective amount may be from 50 to 95 wt%, alternatively from 50 to 80 wt% based on total weight of (1) and the phosphorous pentoxide/methanesulfonic acid reagent. Alternatively, the effective amount of the P₂O₅/H₃CSO₃H reagent may be from 1 to 10 mole equivalents (mol equiv.), alternatively 1 to 5 mol equiv., alternatively 1 to 3 mol equiv. relative to the number of moles of compound (1). E.g., if 1.0 mole of compound (1) is used in the contacting step (A), then the effective amount of the P₂O₅/H₃CSO₃H reagent may be from 1 to 10 moles, alternatively 1 to 5 moles, alternatively 1 to 3 moles.

Hydride-functional reducing agent means a compound having a metal-H bond capable of adding to an oxo group of a ketone to give a tertiary alcohol. Suitable metals include Al and B. Suitable hydride-functional reducing agents are lithium aluminum hydride (LiAlH₄), diisobutyl aluminum hydride (i-Bu₂AlH), and sodium borohydride (NaBH₄).

Methanesulfonic acid is a compound of formula H₃CSO₃H and has CAS number 75-75-2 and is widely available from commercial suppliers.

Phosphorous pentoxide is a compound of formula P₂O₅ (also written as P₄O₁₀) and has CAS number 1314-56-3 and is widely available from commercial suppliers.

The phosphorous pentoxide and methanesulfonic acid reagent ("P₂O₅/H₃CSO₃H reagent", also written as P₄O₁₀/H₃CSO₃H reagent or P₂O₅/MeSO₃H reagent) is a physical blend of P₂O₅ (also written as P₄O₁₀) and H₃CSO₃H, or a reaction product thereof. The weight/weight ratio of P₂O₅/H₃CSO₃H in the reagent may be from 0.05/1 to 1/1, alternatively 0.1/1 to 1/1 alternatively 0.15/1 to 1/1, alternatively 0.2/1 to 1/1, alternatively 0.05/1 to 0.14/1, alternatively 0.1/1. The 0.1/1 (wt/wt) P₂O₅/H₃CSO₃H reagent is commercially available and may be referred to as Eaton's reagent. The reagent of P₂O₅ and CH₃SO₃H may be formed *in situ* in the presence of the compound (1), such as prior to or during the contacting step (A). Alternatively, the reagent of P₂O₅ and CH₃SO₃H may be pre-formed (in absence of compound (1)) before contacting step (A). It is convenient to pre-form the P₂O₅/CH₃SO₃H reagent before contacting step (A), and store the resulting preformed reagent for later use in embodiments of the contacting step (A). In some aspects the method further comprises limitation (i) or (ii): (i) a step of preforming the P₂O₅/H₃CSO₃H reagent before the contacting step (A) and in the absence of compound (1); or (ii) wherein the contacting step further comprises contacting a phosphorous pentoxide and methanesulfonic acid together in the presence of compound (1) to form the P₂O₅/H₃CSO₃H reagent *in situ.*

Polyphosphoric acid or PPA has CAS no. 8017-16-1 and is a compound generally of formula HO-[P(=O)(OH)]ₙ-H, wherein subscript n indicates degree of polymerization. PPA consists of oxygen and phosphorous atoms and is free of sulfur and carbon atoms.

In some aspects each reactant, reagent, solvent, or other material used in the inventive methods, and each product thereof, is free of Pt, Ni, Pd, Rh, and Ru.

Under reaction conditions sufficient to make are included reaction temperature; reaction pressure; reaction atmosphere; reaction solvent, if any; reactant and reagent concentrations; molar ratios of reactants to each other and to reagents; and absence of negating compounds. Reaction pressure is typically room pressure (e.g., 101 kilopascals (kPa), except higher for olefin polymerization reactions. If desired reactions (e.g., steps (A) to (F)) may be carried out in a fume hood under an anhydrous molecular nitrogen gas atmosphere or using Schlenck line techniques and conditions.

Reaction temperatures under reaction conditions sufficient to make may vary from step to step. For example, in step (A) (cyclization), the under reaction conditions sufficient to make compound (2) may include a reaction temperature of from -78° to 30° C., alternatively from -30° to 25° C., alternatively from 0° to 25° C., alternatively from -5° to 5° C. In steps (B) (hydride reduction or alkyl lithium addition), (D) (deprotonation of a cyclopentadiene), (E) (forming a substituted metallocene compound) and (F) (forming a zirconocene dimethyl) the reaction temperatures may be independently from -30° to 110° C., alternatively from 0° to 50° C., alternatively from 10° to 30° C. In step (C) (dehydration) the reaction temperature may be from 0° to 120° C., alternatively from 20° to 110° C., alternatively from 30° to 100° C.

The use or not of solvent and the type of solvent if used under reaction conditions sufficient to make may vary from step to step. Step (A) may be free of solvent or may employ a solvent. When the amount of the methanesulfonic acid is sufficient for solubilizing reactants, a solvent may be omitted. Alternatively, polar aprotic solvent may be employed. The polar aprotic solvent may be selected from sulfolane, 1,2-dimethoxyethane, 1-methoxy-2-(2-methoxyethoxy)ethane, and mixtures of any two or more thereof. The amount of polar aprotic solvent employed is not particularly important. The foregoing polar aprotic solvents may serve to solubilize the compound (1) and/or the P₂O₅/H₃CSO₃H reagent. The amount of solvent employed may be sufficient to prepare a starting solution that is from 0.5 Molar (M) to 5 M, or 1 M to 2.5 M of P₂O₅/H₃CSO₃H reagent therein. The polar aprotic solvent may allow the contacting step (A) to be performed at lower temperatures within the ranges given above therefor. A polar aprotic solvent is used for the P₂O₅/H₃CSO₃H reagent because a protic solvent is expected to undesirably react with the P₂O₅/H₃CSO₃H reagent, which is a powerful dehydrating agent. The polar aprotic solvent may be of intermediate polarity in order to co-solubilize the compound (1) and P₂O₅/H₃CSO₃H reagent. The polar aprotic solvent may be capable of producing a homogeneous solution of the compound (1) at from -25º to 25º C., alternatively at 25º C.,alternatively at 10º C., alternatively at 0º C., alternatively at -10º C., alternatively at -25º C. A homogeneous solution is not required for successful reaction of compound (1) in the presence of the P₂O₅/H₃CSO₃H reagent. In steps (B) (hydride reduction or alkyl lithium addition), (D) (deprotonation of a cyclopentadiene), (E) (forming a substituted metallocene compound) and (F) (forming a zirconocene dimethyl) an anhydrous, non-polar aprotic solvent such as an alkyl ether such as diethyl ether, tetrahydrofuran, or dioxane may be used. In step (B) when the hydride-functional reducing agent is used and is lithium aluminum hydride or diisobutyl aluminum hydride, the anhydrous, non-polar solvent is used. In step (B) when the hydride-functional reducing agent is used and is sodium borohydride, a polar protic solvent may be used such as methanol, ethanol, 2-propanol, or 1-methoxy-2-(2-methoxyethoxy)ethane. The alkyl lithium reagent may be dissolved in anhydrous alkane solvent such as hexanes, hexane, or heptane. Grignard reagents such as methyl magnesium bromide may be dissolved in an alkyl ether such as dialkyl ether.

Reaction atmosphere included under reaction conditions sufficient to make may be anhydrous molecular nitrogen gas or Schlenck line conditions for step (A) (cyclization) and air for step (C) (dehydrating). Reaction atmosphere for step (B) (hydride reduction or alkyl lithium addition), (D) (deprotonation of a cyclopentadiene), (E) (forming a substituted metallocene compound) and (F) (forming a zirconocene dimethyl) may be an inert gas such as anhydrous nitrogen, argon or helium gas, or a mixture of any two or more thereof.

Reaction concentrations of reactants and reagents included under reaction conditions sufficient to make may be independently in the range from 0.1 to 1.4 M, alternatively 0.25 to 1 Molar (M), alternatively 0.4 to 1 M.

Molar ratios of reactants to each other and to reagents included under reaction conditions sufficient to make may vary from 0.25 times to 1.5 times theoretical reaction stoichiometry, alternatively from 0.99 times to 1.2 times theoretical reaction stoichiometry, alternatively from 1.0 to 1.1 times theoretical reaction stoichiometry, depending upon the reactants and reagents used. In step (B) (hydride reduction or alkyl lithium addition), the theoretical reaction stoichiometry of the hydride-functional reducing agent to compound (2) is 0.25 LiAlH4 or NaBH4 to 1.0 compound (2) and 0.5 i-Bu2AlH to 1.0 compound (2) and 1.0 (C₁-C₄)alkyl lithium to 1.0 compound (2). The theoretical reaction stoichiometry for step (C) (dehydration) is catalytic in acid catalyst up to, typically, 1:1. The theoretical reaction stoichiometry for each of steps (D) (deprotonation of a cyclopentadiene), or (E) (forming a substituted metallocene compound) is typically 1:1. The theoretical reaction stoichiometry for step (F) (forming a zirconocene dimethyl) is 2.0 methyl magnesium bromide to 1.0 compound (7).

Negating agents should not be included under reaction conditions sufficient to make. In step (A) (cyclization), a negating agent may be a quantity of a basic compound that would neutralize the acidity of the P₂O₅/H₃CSO₃H reagent or otherwise render it ineffective. For example, purity of compound (1) used in step (A) may be at least 95%, alternatively at least 98%, alternatively at least 99%, alternatively at least 99.5% by weight. In steps (B) (hydride reduction or alkyl lithium addition), (D) (deprotonation of a cyclopentadiene), (E) (forming a substituted metallocene compound) and (F) (forming a zirconocene dimethyl), a negating agent would be a protic compound (e.g., a NH functional, OH functional, and/or SH functional compound) or an oxidizing agent. Examples of NH functional compounds are primary and secondary amines and amides. Examples of OH functional compounds are alcohols, carboxylic acids, and oximes. Examples of SH functional compounds are thiols (mercaptans). Examples of NH and OH functional compounds are primary and secondary amino alcohols and amino acids. In step (C) (dehydrating), a negating agent would be added water (not counting water formed as a by-product of the dehydrating step) or a quantity of a basic compound that would neutralize an acid dehydration catalyst used therein. Purity of compound (2) used in step (b), compound (3) used in step (C), compound (4) used in step (D), compound (6) used in step (D), and compound (7) used in step (F) independently may be at least 95%, alternatively at least 98%, alternatively at least 99%, alternatively at least 99.5% by weight.

A compound includes all its isotopes and natural abundance and isotopically-enriched forms. The enriched forms may have medical or anti-counterfeiting uses.

In some aspects any compound, composition, formulation, mixture, or reaction product herein may be free of any one of the chemical elements selected from the group consisting of: H, Li, Be, B, C, N, O, F, Na, Mg, Al, Si, P, S, Cl, K, Ca, Sc, Ti, V, Cr, Mn, Fe, Co, Ni, Cu, Zn, Ga, Ge, As, Se, Br, Rb, Sr, Y, Zr, Nb, Mo, Tc, Ru, Rh, Pd, Ag, Cd, In, Sn, Sb, Te, I, Cs, Ba, Hf, Ta, W, Re, Os, Ir, Pt, Au, Hg, Tl, Pb, Bi, lanthanoids, and actinoids; with the proviso that chemical elements required by the compound, composition, formulation, mixture, or reaction product (e.g., C and H required by a polyolefin or C, H, and O required by an alcohol) are not excluded.

The following apply unless indicated otherwise. Alternatively precedes a distinct embodiment. ASTM means the standards organization, ASTM International, West Conshohocken, Pennsylvania, USA. Any comparative example is used for illustration purposes only and shall not be prior art. Free of or lacks means a complete absence of; alternatively not detectable. May confers a permitted choice, not an imperative. Operative means functionally capable or effective. Optional(ly) means is absent (excluded), alternatively is present (included). Periodic Table of the Elements is the IUPAC version dated May 1, 2013. Properties are measured using a standard test method and conditions for the measuring (e.g., viscosity: 23° C and 101.3 kPa). Ranges include endpoints, subranges, and whole and/or fractional values subsumed therein, except a range of integers does not include fractional values. Room temperature: 23° C. ± 1° C. Substituted when referring to a compound means having, in place of hydrogen, one or more substituents, up to and including per substitution. Any conflict between structure and name of a compound, the structure controls.

### EXAMPLES

Unless noted otherwise herein, use the following preparations for characterizations. Carry out syntheses under an atmosphere of dry nitrogen in a glovebox when indicated. Perform reactions requiring anhydrous conditions under an atmosphere of dry nitrogen in oven-dried glassware cooled under a stream of dry nitrogen. Anhydrous toluene, hexanes, tetrahydrofuran, diethyl ether and 1,2-dimethoxyethane are from Sigma-Aldrich. Solvents that are used for experiments performed in a nitrogen-filled glovebox are further dried by storage over activated 0.4 nm (4 Angstrom (Å)) molecular sieves. Cyclopentadienylzirconium (IV) chloride (compound (6a), which is compound (6) wherein M is Zr and R6-R10 is H, "(Cp)ZrCl₃") is purchased from Boulder Scientific and is used as received. All other reagents are purchased from Sigma-Aldrich and are used as received. For example, 0.1/1 (wt/wt) P₂O₅/MeSO₃H reagent may be purchased from Sigma-Aldrich CAS# 39394-84-8.

¹H-NMR (proton nuclear magnetic resonance spectroscopy) chemical shift data are reported in parts per million (ppm) down field relative to tetramethylsilane (TMS), δ scale, using residual protons in deuterated solvent as references. The ¹H-NMR chemical shift data measured in CDCl₃ are referenced to 7.26 ppm, data measured in benzene-d6 (C₆D₆) to 7.16 ppm, data measured in tetrahydrofuran-d8 (THF-d8) to 3.58 ppm. ¹H-NMR chemical shift data are reported in the format: chemical shift in ppm (multiplicity, coupling constant(s) in Hertz (Hz), and integration value. Multiplicities are abbreviated s (singlet), d (doublet), t (triplet), q (quartet), pent (pentet), m (multiplet), and br (broad).

GC/MS (EI) means gas chromatography (electron ionization). The following units are used in the examples and 1 inch = 0.0254 m, 1 foot = 0.3048 m and 1 oz = 29.5735 ml.

Preparation 1: synthesis of (1a) (2E)-2-butenoic acid, cyclohexyl ester 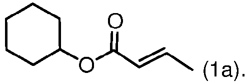 In a fume hood, cyclohexanol (30 milliliters (mL), 283.9 millimoles (mmol)), crotonic acid (25.9 g, 300.98 mmol), p-toluene sulfonic acid (1.08 g, 5.68 mmol), and 40 mL of toluene were charged into a 250 mL round bottom flask. The flask was equipped with a Dean-Stark trap and a reflux condenser. The resulting reaction mixture was heated to reflux, and water that was generated was removed azeotropically. After refluxing for 18 hours, the reaction mixture was cooled to ambient temperature, and quenched with water (55 mL). The resulting organic layer was separated and washed with saturated aqueous NaHCO₃ (2 x 40 mL), then brine (30 mL), and then dried over magnesium sulfate and filtered. The solvent was removed under reduced pressure to give compound (1a) as a light yellow liquid (40.6 g) in 85% yield. ¹HNMR (400 MHz, Chloroform-d) δ 6.93 (dq, 1H), 5.81 (dq, 1H), 4.84 - 4.73 (m, 1H), 1.92 - 1.80 (m, 4H), 1.77 - 1.64 (m, 3H), 1.59 - 1.12 (m, 6H) and GC/MS (EI) (Mass found = 168, 87, 69) were consistent with (2E)-2-butenoic acid, cyclohexyl ester.

Preparation 2 (prophetic): synthesis of (1b) (2E)-2-butenoic acid, 3,5,7-trimethylcyclooctyl ester (1e). Replicate Preparation 1 except substitute 284 mmol of (a12) 3,5,7-trimethylcyclooctanol (CAS 1823711-29-0) for the cyclohexanol to give (2E)-2-butenoic acid, 3,5,7-trimethylcyclooctyl ester (1b).

Preparation 3: synthesis of (2E)-2-butenoic acid, cyclopentyl ester. 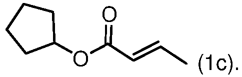 In a fume hood, into a 50 mL round bottom flask equipped with a Dean-Stark trap and a reflux condenser, charge cyclopentanol (2.1 mL, 23.2 mmol), crotonic acid (2.11 g, 24.6 mmol), p-toluene sulfonic acid (0.088 g, 0.46 mmol), and 5 mL of toluene. Heat the resulting mixture to reflux, and remove generated water azeotropically. After refluxing for 18 hours, cool the reaction mixture to ambient temperature, and quench with water (10 mL). Separate the resulting organic layer, and wash it with saturated aqueous NaHCO₃ (2 x 10 mL), then brine (20 mL), and then dry over magnesium sulfate. Filter and remove solvent from the filtrate under reduced pressure to give 2.8 g (78% yield) of compound (1c) as a colorless liquid. ¹HNMR and GC/MS (Mass found = 154) were consistent with (2E)-2-butenoic acid, cyclopentyl ester. Compound (1c) is characterized by GC/MS (EI) 154 (mass), 87, 69.¹H NMR (400 MHz, Chloroform-d) δ 6.91 (dq, 1H), 5.79 (dq, 1H), 5.18 (tt, 1H), 1.94 - 1.79 (m, 5H), 1.83 - 1.63 (m, 4H), 1.67 - 1.48 (m, 2H).

Inventive Example 1: synthesis of compound (2a): 2,3,4,5,6,7-hexahydro-3-methyl-1H-inden-1-one 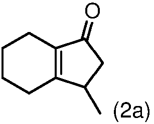 (compound (2) wherein subscript n is 2, R1-R3 is H and R4 is methyl). In a fume hood, under a nitrogen atmosphere in a 250 mL round bottom flask equipped with a stir bar, added compound (1a) (2E)-2-butenoic acid, cyclohexyl ester (3 g, 17.8 mmol). Cooled the ester in the flask to 0° C. Then added dropwise P₂O₅/H₃CSO₃H reagent (0.1/1)) (8.49 mL, 53.5 mmol) at 0° C. Warmed the resulting reaction mixture with stirring to ambient temperature (23° C.), and continued stirring for 72 hours at ambient temperature. Diluted the resulting crude product with 20 mL of water, then added solid NaHCO₃ in portions until bubbling subsided to give quenched mixture having pH 8 to pH 9. Separated aqueous and organic layers of quenched mixture in a separatory funnel. Extracted the aqueous layer three times with diethyl ether (3 × 20 mL). Combined the organic layer with the three extracts, and washed the combination with brine (30 mL), dried over magnesium sulfate, and filtered. Removed solvent *in vacuo* to give 2.45 g of compound (2a) as a light brown oil product (91.4 % yield). ¹HNMR (400 MHz, Chloroform-d) δ 2.77 - 2.67 (m, 1H), 2.61 (ddd, 1H), 2.48 - 2.34 (m, 1H), 2.32 - 2.03 (m, 3H), 2.03 - 1.47 (m, 5H), 1.14 (d, 3H) was consistent with (2a) 2,3,4,5,6,7-hexahydro-3-methyl-1H-inden-1-one.

Inventive Example 2 (prophetic): synthesis of compound (3a) (compound (3) wherein subscript n is 2, R1 to R3 is H and each of R4 and R5 is methyl). Under an atmosphere of dry nitrogen, weigh out the compound (2a) of Inventive Example 1 (136 mmol) in a 500 mL round bottom flask, and dissolve in anhydrous diethyl ether (245 mL). Cool the reaction mixture to -78 °C. Add dropwise methyl lithium (1.6 M, 110 mL, 176.3 mmol, metal-R5 reducing agent wherein R5 is methyl), and stir the solution for 15 minutes at -78° C. Stir the reaction mixture for 20 hours at room temperature to give a reaction mixture containing compound (3a). Compound (3a) may be isolated and/or characterized by ¹H-NMR and/or GC-MS if desired.

Inventive Example 3 (prophetic): synthesis of compound (4a) (compound (4) wherein subscript n is 2, R1 to R3a is H and each of R4 and R5 is methyl). Add aqueous 6 M HCL (67 mL) to the reaction mixture containing compound (3a) in Inventive Example 2, and hydrolyze with stirring for 20 hours at room temperature. Separate the organic phase. Extract the aqueous layer with diethyl ether (2 x 50 mL). Combine organic layers, and wash with water (80 mL), then saturated NaHCO₃ (80 mL), and then brine (80 mL). Dry the organic layers over magnesium sulfate and filter, Remove the solvent in vacuo to give compound (4a) as an orange liquid. Compound (4a) may be isolated and/or characterized by ¹H-NMR and/or GC-MS if desired.

Inventive Example 4 (prophetic): synthesis of compound (5a) (compound (5) wherein subscript n is 2, R1 to R3a is H each of R4 and R5 is methyl). In a glove box, in a 16-oz glass jar, dissolve compound (4a) (50 mmol) in hexanes (140 mL). To the stirred solution add dropwise a solution of n-butyl lithium in hexanes (1.6 M, 46.6 mL, 74.5 mmol). Stir the reaction mixture for 20 hours. Collect the compound (5a) by vacuum filtration, and wash the resulting solid product with hexanes. Dry under vacuum to give compound (5a) as a solid. Compound (5a) may be isolated and/or characterized by ¹H-NMR if desired.

Inventive Example 5 (prophetic): synthesis of compound (7a) (compound (7) wherein subscript n is 2, R1 to R3a and R6 to R10 are H and each of R4 and R5 is methyl and M is Zr). In drybox in a 32-oz glass jar, slurry compound (5a) (31 mmol) in 272 mL of anhydrous diethyl ether. To the stirred reaction mixture add (Cp)ZrCl₃ (8.12 g, 31.1 mmol, compound (6a)) in small portions, then add 1,2-dimethoxyethane (27 mL). Stir the resulting dark orange reaction mixture for 48 hours at room temperature, filter, and remove the solvent under vacuum to give compound (7a). Compound (7a) may be isolated and/or characterized by ¹H-NMR if desired.

Inventive Example 6 (prophetic): synthesis of compound (8a) (compound (8) wherein subscript n is 2, R1 to R3a and R6 to R10 are H and each of R4 and R5 is methyl). In drybox in an 8-oz glass jar, slurry compound (7a) (10.5 mmol) in anhydrous diethyl ether (65 mL). To the stirred reaction mixture add dropwise a solution of methyl magnesium bromide (3.0 M, 7.89 mL, 23.7 mmol). Stir the reaction mixture for 20 hours at room temperature. Remove the solvent under vacuum. Dissolve the resulting solid product in hexanes (150 mL) and filter. Remove the hexanes under vacuum to give compound (8a). Compound (8a) may be isolated and/or characterized by 1H-NMR if desired.

Inventive Example 7: (prophetic) polymerization of ethylene using a catalyst prepared from compound (7a) or (8a) to give a polyethylene polymer. Use a gas-phase fluidized bed reactor ("Reactor") having a reaction zone dimensioned as 304.8 mm (twelve inch) internal diameter and a 2.4384 meter (8 feet) in straight-side height and containing a fluidized reactor bed of polymer granules. Configure the Reactor with a recycle gas line for flowing a recycle gas stream. Fit the Reactor with gas feed inlets, catalyst inlet, and polymer product outlet. Feed catalyst prepared from compound (7a) of Inventive Example 5 or from compound (8a) of Inventive Example 6 into the fluidized bed reactor. Introduce gaseous feed streams of ethylene and hydrogen together with liquid 1-hexene comonomer below the fluidized reactor bed into the recycle gas line. Control individual flow rates of ethylene ("C2"), hydrogen ("H2") and 1-hexene ("C6") to maintain a fixed 1-hexene comonomer to ethylene monomer composition molar ratio ("C6/C2") from 0.0001 to 0.1 (e.g., 0.0050), a constant hydrogen to ethylene molar ratio ("H2/C2") from 0.0001 to 0.1 (e.g., 0.0020), and a constant ethylene ("C2") partial pressure from 1,000 to 2,000 kilopascals (kPa) (e.g., 1,500 kPa). Measure concentrations of all gases by an in-line gas chromatograph to ensure relatively constant composition in the recycle gas stream. Maintain a reacting bed of growing polymer particles in a fluidized state by continuously flowing a make-up feed and recycle gas through the reaction zone. Use a superficial gas velocity of from 0.4 to 0.7 meter per second (m/sec) (e.g., from 0.49 to 0.67 m/sec, or 1.6 to 2.2 feet per second (ft/sec)). Operate the Reactor at a total pressure of 2,000 to 3,000 kPa (e.g., 2344 to about 2413 kPa, or 340 to about 350 pounds per square inch-gauge (psig)) and at a constant reaction temperature of 85° to 115° C. (e.g., 105° C.). Maintain the fluidized bed at a constant height by withdrawing a portion of the bed at a rate equal to the rate of formation of particulate product. The foregoing process yields a product comprising a polyethylene polymer. The polymer production rate is in the range of 5 to 20 kg/hour (e.g., 13 to 18 kg/hour. Remove the polymer product semi-continuously via a series of valves into a fixed volume chamber, wherein this removed polymer product is purged to remove entrained hydrocarbons and treated with a stream of humidified nitrogen (N₂) gas to deactivate any trace quantities of residual polymerization catalyst.

**Table 1: prophetic polyethylene made using catalyst from compound (7a) or (8a).**

| **Property** | **Expected Inventive Result** |
|---|---|
| Catalyst mass balance productivity (wt/wt) | 5,000 |
| Melt index I2 (190 C., 2.16 kg, ASTM D1238-13) | 70 grams/10 minutes |
| Density (ASTM D792-13, Method B) | 0.96 g/cm3 |
| Butyl branching frequency* (BBF, NMR) | 0.6 |
| Number average molecular weight | 10,000 g/mole |
| Weight average molecular weight | 40,000 g/mole |
| Molecular mass dispersity (M_{w}/Mₙ), Ð_{M}, | 3 |
| Melting temperature Tₘ | 130 |

*BBF is the number of butyl branches per 1000 main chain carbon atoms.

As can be seen from Table 1 the polymerization catalyst produced in Inventive Example 7 is expected to have a desired catalytic activity and a resultant polyethylene polymer having desirable properties.

Inventive Example 8 (prophetic): synthesis of compound (2b) bicyclo[3.3.0]-1(5)-octen-2-one 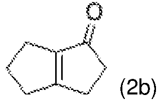 (compound (2) wherein subscript n is 1 and R1-R4 are H). Replicate Inventive Example 1 except substitute 18 mmol of (1b) propenoic acid, cyclopentyl ester (CAS 16868-13-6) for compound (1a) to give compound (2b).

Inventive Example 9: synthesis of compound (2c) (4-methyl-bycyclo[3.3.0]-1(5)-octen-2-one, i.e., compound (2) wherein subscript n is 1 and R1-R3 are H and R4 is methyl): 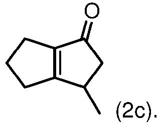 In a fume hood, under a nitrogen atmosphere in a 100 mL round bottom flask equipped with a stir bar, added compound (1c) (2E)-2-butenoic acid, cyclopentyl ester (0.5 g, 3.24 mmol) of Preparation 3. Cooled the ester in the flask to 0° C. Then added dropwise P₂O₅/H₃CSO₃H reagent (0.1/1)) (1.5 mL, 9.73 mmol) at 0° C. Warmed the resulting reaction mixture with stirring to ambient temperature (23° C.), and continued stirring for 72 hours. Diluted the resulting crude product with 5 mL of water, then added solid NaHCO₃ in portions until bubbling subsided to give quenched mixture having pH 8 to pH 9. Separated aqueous and organic layers of quenched mixture in a separatory funnel. Extracted the aqueous layer three times with diethyl ether (3 × 8 mL). Combined the organic layer with the three extracts, and washed the combination with brine (15 mL), dried over magnesium sulfate, and filtered. Removed solvent *in vacuo* to give 0.42 g (95 % yield) of compound (2c) as an orange oil. ¹H NMR (400 MHz, Chloroform-d) δ 3.05 - 2.89 (m, 1H), 2.81 (dt, 1H), 2.66 - 2.47 (m, 1H), 2.47 - 2.26 (m, 6H), 1.19 (d, 3H) was consistent with compound (2c).

Inventive Example 10 (prophetic): synthesis of compound (2d) 10-methyl-bicyclo[5.3.0]-1(7)-decen-8-one (compound (2) wherein subscript n is 3 and R1-R3 are H and R4 is methyl). Replicate Inventive Example 1 except substitute 18 mmol of (1c) (2E)-2-butenoic acid, cycloheptyl ester (CAS 10555-39-2) for compound (1a) to give compound (2d).

Inventive Example 11 (prophetic): synthesis of compound (2e) 2,4,6,11-tetramethyl-bicyclo[6.3.0]-1(8)-undecen-9-one (compound (2) wherein subscript n is 4 and R1-R4 are methyl). Replicate Inventive Example 1 except substitute 18 mmol of (1e) (2E)-2-butenoic acid, 3,5,7-trimethylcyclooctyl ester (Preparation 2) for compound (1a) to give compound (2e).

Inventive Example 12 (prophetic): synthesis of compounds (3b) to (3e) (compounds (3) wherein subscript n and groups R1-R4 are as defined for compounds (2b) to (2e), respectively). Replicate Inventive Example 2 except replace compound (2a) with any one of compounds (2b) to (2e) to give a reaction mixture containing any one of compounds (3b) to (3e), respectively, wherein subscript n and groups R1-R4 are as defined for compounds (2b) to (2e), respectively, and R5 is methyl. Compound (3b) to (3e) may be isolated and/or characterized by 1H-NMR and/or GC-MS if desired.

Inventive Example 13 (prophetic): synthesis of compounds (4b) to (4e). Compound (4b) is compound (4) wherein subscript n is 1 and R1-R4 are H and R5 is methyl. Compound (4c) is compound (4) wherein subscript n is 1 and R1-R3A are H and each of R4 and R5 is methyl. Compound (4d) is compound (4) wherein subscript n is 3 and R1-R3A are H and each of R4 and R5 is methyl. Compound (4e) is compound (4) wherein subscript n is 4 and R1, R1A, two R2, and each R2A are H and two R2 and each of R3, R4 and R5 is methyl. Replicate Inventive Example 3 except replace compound (3a) with any one of compounds (3b) to (3e), respectively, to give a reaction mixture containing any one of compounds (4b) to (4e), respectively, wherein subscript n and groups R1-R5 are as defined for compounds (3b) to (3e), respectively. Compound (4b) to (4e) may be isolated and/or characterized by 1H-NMR and/or GC-MS if desired.

Inventive Example 14 (prophetic): synthesis of compounds (5b) to (5e) (compound (5) wherein subscript n and groups R1-R5 are as defined for compounds (4b) to (4e), respectively). Replicate Inventive Example 4 except replace compound (4a) with any one of compounds (4b) to (4e), respectively, to give a reaction mixture containing any one of compounds (5b) to (5e), respectively, wherein subscript n and groups R1-R5 are as defined for compounds (4b) to (4e), respectively. Compound (5b) to (5e) may be isolated and/or characterized by ¹H-NMR if desired.

Inventive Example 15 (prophetic): synthesis of compounds (7b) to (7e) (compound (7) wherein subscript n and groups R1-R5 are as defined for compounds (5b) to (5e), respectively, and R6 to R10 are H and M is Zr). Replicate Inventive Example 5 except replace compound (5a) with any one of compounds (5b) to (5e), respectively, to give a reaction mixture containing any one of compounds (7b) to (7e), respectively, wherein subscript n and groups R1-R5 are as defined for compounds (5b) to (5e), respectively, and R6 to R10 are H and M is Zr. Compound (7b) to (7e) may be isolated and/or characterized by ¹H-NMR if desired.

Inventive Example 16 (prophetic): synthesis of compounds (8b) to (8e) (compound (8) wherein subscript n, groups R1 to R10, and M are as defined for compounds (7b) to (7e), respectively). Replicate Inventive Example 6 except replace compound (7a) with any one of compounds (7b) to (7e), respectively, to give a reaction mixture containing any one of compounds (8b) to (8e), respectively, wherein subscript n, groups R1 to R10, and M are as defined for compounds (7b) to (7e), respectively. Compound (8b) to (8e) may be isolated and/or characterized by ¹H-NMR if desired.

Inventive Example 17: (prophetic) polymerization of ethylene using a catalyst prepared from any one of compounds (7b) to (7e) or from any one of compounds (8b) to (8e). Replicate Inventive Example 7 except replace compound (7a) with any one of compounds (7b) to (7e), or replace compound (8a) with any one of compounds (8b) to (8e), to give a different product comprising a polyethylene polymer that is different than the polyethylene polymer made by Inventive Example 7.

As discussed earlier, Conia et al., Rand and Dolinski, and others report using PPA or P₂O₅/PPA mixture to catalyze a reaction of cycloheptene, cyclohexene, or cyclopentene with an alpha,beta-unsaturated carboxylic acid such as acrylic acid or crotonic acid gives a reaction mixture that contains an ester by-product (e.g., cycloheptyl crotonate, cyclohexyl crotonate, or cyclopentyl crotonate, respectively). We found that using the phosphorous pentoxide/methanesulfonic acid reagent to catalyze a reaction of cycloheptene, cyclohexene, or cyclopentene with an alpha, beta-unsaturated carboxylic acid such as acrylic acid or crotonic acid gives a reaction mixture that does not contain an ester by-product (e.g., the reaction does not yield cycloheptyl crotonate, cyclohexyl crotonate, or cyclopentyl crotonate, respectively). We base this finding on analysis of at least one of the reaction mixtures by gas chromatography-mass spectrometry (GC-MS), which fails to show any ester by-product. We also base this finding on seeing that a reaction of cycloheptene, cyclohexene, or cyclopentene with an alpha, beta-unsaturated carboxylic acid such as acrylic acid or crotonic acid in the presence of the P₂O₅/H₃CSO₃H reagent goes much faster than the reaction of cycloheptyl crotonate, cyclohexyl crotonate, or cyclopentyl crotonate, respectively, in the presence of the P₂O₅/H₃CSO₃H reagent.

Without wishing to be bound by theory, we believe that the P₂O₅/H₃CSO₃H reagent reacts with the alpha,beta-unsaturated carboxylic acid (e.g., crotonic acid) to give *in situ a* mixed anhydride of general formula R4CH=CHC(=O)-O-SO₂-CH₃, which generates *in situ* an acylium ion (i.e., acyl carbonium ion) of formula R4CH=CHC⁺(=O), which rapidly undergoes a Friedel-Crafts acylation of cycloalkene to give *in situ* a ketone of formula R^{a}-C(=O)-R^{c}, wherein R^{a} is R4CH=CH- and R^{c} is cycloalken-1-yl, which ketone undergoes cyclization reaction to give the corresponding cyclopentenone. For example, when the cycloalkene is cyclohexene and the alpha,beta-unsaturated carboxylic acid is crotonic acid, we believe that the P₂O₅/H₃CSO₃H reagent reacts with the crotonic acid to give *in situ a* mixed anhydride of general formula H₃CCH=CHC(=O)-O-SO₂-CH₃, which generates *in situ* an acylium ion (i.e., acyl carbonium ion) of formula H₃CCH=CHC⁺(=O), which rapidly undergoes a Friedel-Crafts acylation of cycloalkene to give *in situ* a ketone of formula R^{a}-C(=O)-R^{c}, wherein R^{a} is H₃CCH=CH- and R^{C} is cyclohexen-1-yl, which ketone undergoes cyclization reaction to give the corresponding cyclopentenone that is 2,3,4,5,6,7-hexahydro-3-methyl-1H-inden-1-one (i.e., 9-methyl-bicyclo[4.3.0]-8-nonen-7-one). Therefore, using the phosphorous pentoxide/methanesulfonic acid reagent in reaction of a cycloalkene such as cycloheptene, cyclohexene, or cyclopentene with an alpha,beta-unsaturated carboxylic acid such as acrylic acid or crotonic acid does not inherently make the ester by-product (e.g., cycloheptyl crotonate, cyclohexyl crotonate, or cyclopentyl crotonate, respectively) reported by Conia et al., Rand and Dolinski, and others using PPA or P₂O₅/PPA mixture.

## Claims

1. A method of synthesizing a substituted cyclopentadiene compound, the method comprising (A) contacting a compound of formula (1) ("compound (1)"): 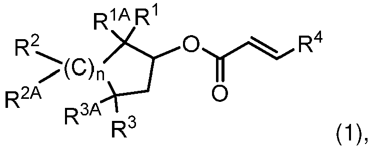 wherein subscript n is 1, 2, 3, or 4; and each of groups R1, R1A, R2, R2A, R3, R3A, and R4 is independently H or (C₁-C₄)alkyl, or any two adjacent R1 to R3 groups are bonded together to form a (C₁-C₄)alkylene and the remaining group of R1 to R3A is H or (C₁-C₄)alkyl, with an effective amount of a phosphorous pentoxide/methanesulfonic acid reagent (P₂O₅/H₃CSO₃H reagent) and under reaction conditions sufficient to make a compound of formula (2) ("compound (2)"): 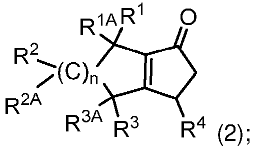 wherein subscript n and groups R1 to R4 are as defined above; and with the proviso that the contacting step (A) is free of a polyphosphoric acid (PPA); (B) contacting the compound (2) with a metal-R5 reducing agent that is either a hydride-functional reducing agent or a (C₁-C₄)alkyl lithium, under reaction conditions sufficient to make a compound of formula (3) ("compound (3)"): 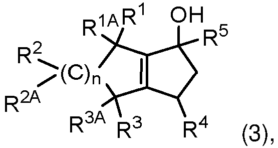 wherein subscript n and groups R1 to R4 are as defined above and R5 is H or (C₁-C₄)alkyl, respectively; and (C) contacting the compound (3) with dehydration reaction conditions to make a substituted cyclopentadiene compound of formula (4) ("compound (4)"): 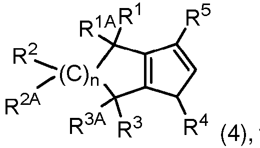 wherein subscript n and groups R1-R5 are as defined above.

2. A method of synthesizing a substituted metallocene compound comprising a metal-(substituted cyclopentadienyl ligand) complex, the method comprising synthesizing the compound (4) according to steps (A) to (C) of claim 1; (D) contacting the compound (4) with an alkyl lithium under reaction conditions sufficient to make a compound of formula (5) ("compound (5)"): 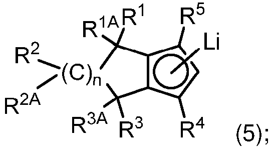 and (E) contacting the compound (5) with a compound of formula (6) ("compound (6)"): 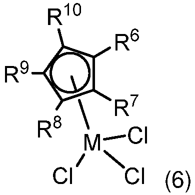 under reaction conditions sufficient to make a compound of formula (7) ("compound (7)"): 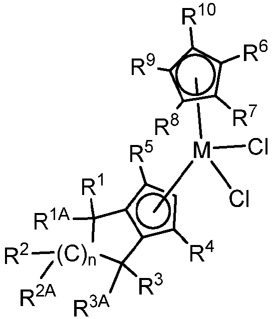 (7) , wherein subscript n and groups R1 to R5 are as defined above; metal M is a metal of Group 4 of the Periodic Table of the Elements; and each of R6 to R8 is independently H or (C₁-C₄)alkyl and R9 and R10 is independently H or (C₁-C₄)alkyl or R9 and R10 are bonded together and are a (C₃-C₅)alkylene.

3. A method of synthesizing a zirconocene dimethyl complex, the method comprising synthesizing the compound (7) according to steps (A) to (E) of claim 2, wherein M is Zr; and (F) contacting the compound (7) with an effective amount of methyl magnesium bromide under reaction conditions sufficient to make a compound of formula (8) ("compound (8)"): 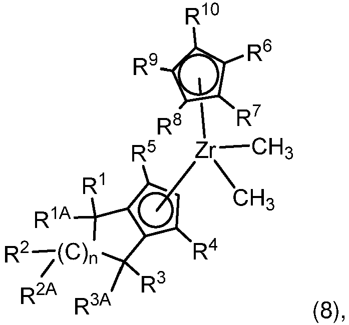 wherein subscript n and groups R1 to R10 are as defined above.

4. The method of any one of claims 1 to 3, wherein the ratio of P₂O₅ to H₃CSO₃H used to make the P₂O₅/H₃CSO₃H reagent is from 0.05/1 to 1/1 (weight/weight).

5. The method of any one of claims 1 to 4, wherein the ratio of P₂O₅ to H₃CSO₃H used to make the P₂O₅/H₃CSO₃H reagent is 0.1/1 (weight/weight).

6. The method of any one of claims 1 to 5, wherein the compound (4) is selected from the group consisting of any one of compounds (4a) to (4e):
compound (4a) is compound (4) wherein subscript n is 2, R1 to R3A is H and each of R4 and R5 is methyl;
compound (4b) is compound (4) wherein subscript n is 1 and R1-R4 are H and R5 is methyl;
compound (4c) is compound (4) wherein subscript n is 1 and R1-R3A are H and each of R4 and R5 is methyl;
compound (4d) is compound (4) wherein subscript n is 3 and R1-R3A are H and each of R4 and R5 is methyl; and
compound (4e) is compound (4) wherein subscript n is 4 and R1, R1A, two R2, and each R2A are H and two R2 and each of R3, R4 and R5 is methyl.

## Patentansprüche

1. Verfahren zum Synthetisieren einer substituierten Cyclopentadienverbindung, das Verfahren umfassend (A) Inberührungbringen einer Verbindung der Formel (1) ("Verbindung (1)"): 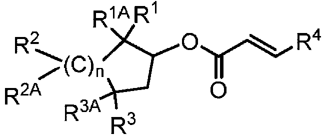 (1), wobei der Index n 1, 2, 3 oder 4 ist; und jede der Gruppen R1, R1A, R2, R2A, R3, R3A und R4 unabhängig H oder (C₁-C₄)-Alkyl ist oder zwei beliebige benachbarte R1 bis R3-Gruppen miteinander gebunden sind, um ein (C₁-C₄)-Alkylen zu bilden, und die verbleibende Gruppe von R1 bis R3A H oder (C₁-C₄)-Alkyl ist, wobei eine wirksame Menge eines Phosphorpentoxid/Methansulfonsäure-Reagens (P₂O₅/H₃CSO₃H-Reagens) und unter Reaktionsbedingungen ausreichend ist, um eine Verbindung der Formel (2) ("Verbindung (2)") herzustellen: 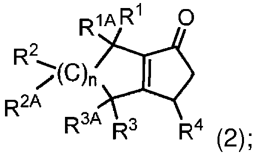 wobei der Index n und die Gruppen R1 bis R4 wie oben definiert sind; und mit der Maßgabe, dass der Kontaktierungsschritt (A) frei von Polyphosphorsäure (PPA) ist; (B) Inberührungbringen der Verbindung (2) mit einem Metall-R5-Reduktionsmittel, das entweder ein hydrid-funktionelles Reduktionsmittel oder ein (C₁-C₄)-Alkyllithium ist, unter Reaktionsbedingungen, die ausreichen, um eine Verbindung von Formel (3) ("Verbindung (3)") herzustellen: 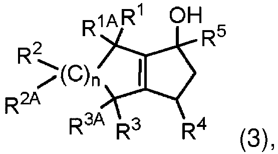 wobei der Index n und die Gruppen R1 bis R4 wie oben definiert sind und R5 jeweils H oder (C1-C4)-Alkyl ist; und (C) Inberührungbringen der Verbindung (3) mit Dehydrationsreaktionsbedingungen, um eine substituierten Cyclopentadienverbindung von Formel (4) ("Verbindung (4)") herzustellen: 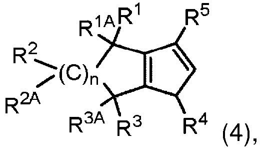 wobei der Index n und die Gruppen R1-R5 wie oben definiert sind.

2. Verfahren zum Synthetisieren einer substituierten Metallocenverbindung, die einen Metall-(substituierter Cyclopentadienylligand)-Komplex umfasst, wobei das Verfahren die Synthese der Verbindung (4) gemäß den Schritten (A) bis (C) von Anspruch 1 umfasst; (D) Inberührungbringen der Verbindung (4) mit einem Alkyllithium unter Reaktionsbedingungen, die ausreichen, um eine Verbindung von Formel (5) ("Verbindung (5)") herzustellen: 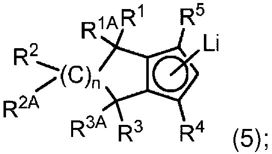 und (E) Inberührungbringen der Verbindung (5) mit einer Verbindung von Formel (6) ("Verbindung (6)"): 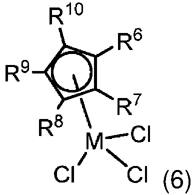 unter Reaktionsbedingungen, die ausreichend sind, um eine Verbindung der Formel (7) ("Verbindung (7)") herzustellen: 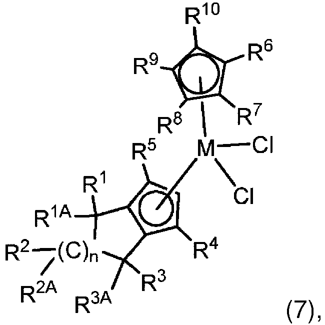 wobei der Index n und die Gruppen R1 bis R5 wie oben definiert sind; Metall M ein Metall der Gruppe 4 des Periodensystems der Elemente ist; und jede von R6 bis R8 unabhängig H oder (C₁-C₄)-Alkyl ist und R9 und R10 unabhängig H oder (C₁-C₄)-Alkyl sind oder R9 und R10 miteinander gebunden sind und ein (C₃-C₅)-Alkylen sind.

3. Verfahren zum Synthetisieren eines Zirconocen-Dimethyl-Komplexes, wobei das Verfahren die Synthese der Verbindung (7) gemäß den Schritten (A) bis (E) von Anspruch 2 umfasst, wobei M Zr ist; und (F) Inberührungbringen der Verbindung (7) mit einer wirksamen Menge an Methylmagnesiumbromid unter Reaktionsbedingungen, die ausreichen, um eine Verbindung der Formel (8) ("Verbindung (8)") herzustellen: 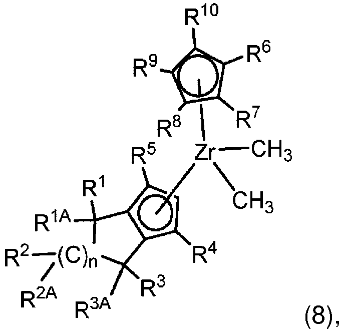 wobei der Index n und die Gruppen R1 bis R10 wie oben definiert sind.

4. Verfahren nach einem der Ansprüche 1 bis 3, wobei das Verhältnis von P₂O₅ zu H₃CSO₃H, das verwendet wird, um das P₂O₅/H₃CSO₃H-Reagens herzustellen, von 0,05/1 bis 1/1 (Gewicht/Gewicht) ist.

5. Verfahren nach einem der Ansprüche 1 bis 4, wobei das Verhältnis von P₂O₅ zu H₃CSO₃H, das verwendet wird, um das P₂O₅/H₃CSO₃H-Reagens herzustellen 0,1/1 (Gewicht/Gewicht) ist.

6. Verfahren nach einem der Ansprüche 1 bis 5, wobei die Verbindung (4) aus der Gruppe ausgewählt ist, bestehend aus einer beliebigen der Verbindungen (4a) bis (4e):
Verbindung (4a) ist Verbindung (4), wobei der Index n 2 ist, R1 bis R3A H ist und jede von R4 und R5 Methyl ist;
Verbindung (4b) ist Verbindung (4), wobei der Index n 1 ist und R1-R4 H sind und R5 Methyl ist;
Verbindung (4c) ist Verbindung (4), wobei Index n 1 ist und R1-R3A H sind und jede von R4 und R5 Methyl ist;
Verbindung (4d) ist Verbindung (4), wobei der Index n 3 ist und R1-R3A H sind und jede von R4 und R5 Methyl ist; und
Verbindung (4e) ist Verbindung (4), wobei der Index n 4 ist und R1, R1A, zwei R2 und jede R2A H sind und zwei R2 und jede von R3, R4 und R5 Methyl ist.

## Revendications

1. Procédé de synthèse d'un composé de cyclopenténone substitué, le procédé comprenant (A) la mise en contact d'un composé de formule (1) (« composé (1) ») : 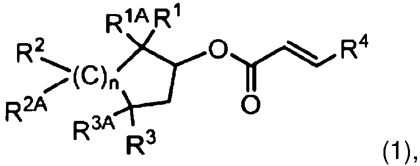 dans lequel l'indice n vaut 1, 2, 3 ou 4 ; et chacun des groupes R1, R1A, R2, R2A, R3, R3A et R4 est indépendamment H ou un alkyle en (C₁ à C₄), ou deux quelconques groupes R1 à R3 adjacents sont liés ensemble pour former un alkylène en (C₁ à C₄) et le groupe restant de R1 à R3A est H ou un alkyle en (C₁ à C₄), avec une quantité efficace d'un réactif de pentoxyde phosphoreux/acide méthanesulfonique (réactif P₂O₅/H₃CSO₃H) et dans des conditions réactionnelles suffisantes pour fabriquer un composé de formule (2) (« composé (2) ») : 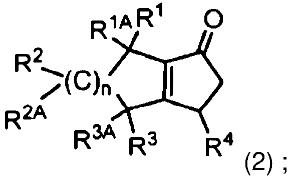 dans lequel l'indice n et les groupes R1 à R4 sont tels que définis ci-dessus ; et à condition que l'étape de mise en contact (A) soit exempte d'acide polyphosphorique (PPA) ; (B) la mise en contact du composé (2) avec un agent réducteur métal-R5 qui est soit un agent réducteur à fonction hydrure soit un lithium d'alkyle en (C₁ à C₄), dans des conditions de réaction suffisantes pour fabriquer un composé de formule (3) (« composé (3) ») : 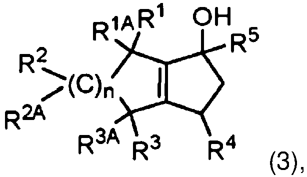 dans lequel l'indice n et des groupes R1 à R4 sont tels que définis ci-dessus et R5 est soit H soit un alkyle en (C₁ à C₄), respectivement ; et (C) la mise en contact du composé (3) avec des conditions de réaction de déshydratation pour fabriquer un composé de cyclopentadiène substitué de formule (4) (« composé (4) ») : 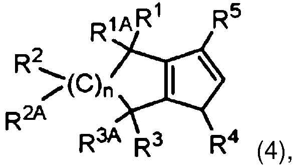 dans lequel l'indice n et les groupes R1 à R5 sont tels que définis ci-dessus.

2. Procédé de synthèse d'un composé métallocène substitué comprenant un complexe métal-(ligand cyclopentadiényle substitué), le procédé comprenant la synthèse du composé (4) selon les étapes (A) à (C) selon la revendication 1 ; (D) la mise en contact du composé (4) avec un lithium d'alkyle dans des conditions de réaction suffisantes pour fabriquer un composé de formule (5) (« composé (5) ») : 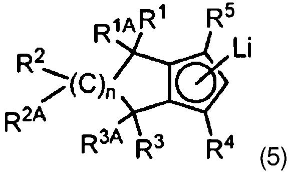 ; et (E) la mise en contact du composé (5) avec un composé de formule (6) (« composé (6) ») : 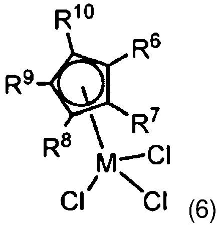 dans des conditions de réaction suffisantes pour fabriquer un composé de formule (7) (« composé (7) ») : 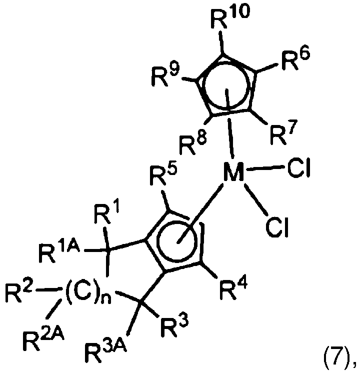 dans lequel l'indice n et les groupes R1 à R5 sont tels que définis ci-dessus ; le métal M est un métal du groupe 4 du tableau périodique des éléments ; et chacun de R6 à R8 est indépendamment H ou un alkyle en (C₁ à C₄) et R9 et R10 sont indépendamment H ou un alkyle en (C₁ à C₄) ou R9 et R10 sont liés ensemble et sont un alkylène en (C₃ à C₅).

3. Procédé de synthèse d'un complexe de diméthyle de zirconocène, le procédé comprenant la synthèse du composé (7) selon les étapes (A) à (E) selon la revendication 2, dans lequel M est Zr ; et (F) la mise en contact du composé (7) avec une quantité efficace de bromure de méthyle magnésium dans des conditions de réaction suffisantes pour fabriquer un composé de formule (8) (« composé (8) ») : 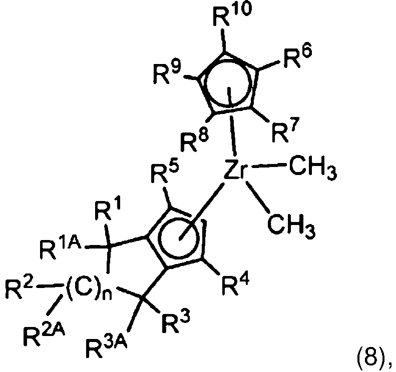 dans lequel l'indice n et les groupes R1 à R10 sont tels que définis ci-dessus.

4. Procédé selon l'une quelconque des revendication 1 à 3, dans lequel le rapport de P₂O₅ à H₃CSO₃H utilisé pour fabriquer le réactif P₂O₅/H₃CSO₃H est de 0,05/1 à 1/1 (poids/poids).

5. Procédé selon l'une quelconque des revendications 1 ou 4, dans lequel le rapport de P₂O₅ à H₃CSO₃H utilisé pour fabriquer le réactif P₂O₅/H₃CSO₃H est de 0,1/1 (poids/poids).

6. Procédé selon l'une quelconque des revendications 1 à 5, dans lequel le composé (4) est choisi dans le groupe constitué par l'un quelconque des composés (4a) à (4e) :
le composé (4a) est le composé (4) dans lequel l'indice n vaut 2, R1 à R3A est H et chacun de R4 et R5 est le méthyle ;
le composé (4b) est le composé (4) dans lequel l'indice n vaut 1 et R1 à R4 sont H et R5 est le méthyle ;
le composé (4c) est le composé (4) dans lequel l'indice n vaut 1 et R1 à R3A sont H et chacun de R4 et R5 est le méthyle ;
le composé (4d) est le composé (4) dans lequel l'indice n vaut 3 et R1 à R3A sont H et chacun de R4 et R5 est le méthyle ; et
le composé (4e) est le composé (4) dans lequel l'indice n vaut 4 et R1, R1A, deux R2, et chaque R2A est H et deux R2 et chacun de R3, R4 et R5 est méthyle.
